# EUROPEAN PATENT APPLICATION

(11) **EP 0 599 419 A2**
(43) Date of publication of application: **01.06.1994**
(21) Application number: 93203259.2
(22) Date of filing: 22.11.1993
(51) Int. Cl.: A61F 2/44, A61F 2/30

(54) **Disk-shaped implant for reinforcing adjacent vertebrae**

(30) Priority: 26.11.1992 DE 9216092 U
(71) Applicant: ESKA Implants GmbH, 23556 Lübeck (DE)
(72) Inventor: Schug Martin, D-23352 Lübeck (DE); Rischke Burkhard, D- 25462 Rellingen (DE)
(74) Representative: Fuchs, Luderschmidt & Partner Patentanwälte

(57) **Abstract**

Disk-shaped implant (2) for reinforcing of adjacent vertebrae characterised in that the implant (2) has a disk shaped core (4) made of solid metal having an open-pored or open-celled metal structure (6) provided on the surfaces of the core (4). The advantage of the invention is that bone tissue is conserved while effective reinforcement is achieved.

## Description

This invention concerns a disk-shaped implant for reinforcing adjacent vertebrae.

Such implants are known in a variety of designs and are used to join neighboring vertebrae securely in order to prevent relative movement of the neighboring vertebrae when the disk is damaged or worn.

European Patent A 271,501 discloses such an implant that consists of a cylindrical block with an open-cell or open-pore surface structure and is implanted into a recess that is resected on the opposing bone surfaces of adjacent vertebrae. The receptacle space necessary for the implant between adjacent vertebrae is approximately adapted to the shape of the implant in order to achieve a form-fitting connection between the neighboring vertebrae which permits a certain primary strength after the surgery until finally the adjacent bone tissue has grown into the open-pore surface structure and joins the adjacent vertebrae together so they cannot twist. A disadvantage of this design is that a relatively large volume of bone tissue must be resected even when the facing load-bearing surfaces of the adjacent vertebrae still show little or no signs of wear.

Therefore, the object of this invention is to develop an implant that will permit an effective reinforcement of adjacent vertebrae in an implantation technique that saves as much bone tissue as possible.

This object is achieved according to this invention with the implant of the aforementioned type by means of a disk-shaped core of solid metal and an open-pore or open cell metal structure on both sides of the core.

The advantages of this invention include especially the fact that the disk-shaped implant has approximately the shape of a natural disk and can be inserted between the adjacent vertebrae - after removing the natural disk - without requiring resection of bone materials. Whereas a primary reinforcement effect is achieved by the vertebral column pressure on the two opposing supporting surfaces, long-term stabilization is achieved through the connective tissue that grows into the open pore or open celled metal structure. Thus, it is possible to implant the implant according to this invention in a procedure that saves bone tissue while at the same time the long-term stability of the reinforcement of the spinal column is achieved by the fact that a bond is created between the open-pore metal structure and the regenerated connective tissue that grows into the pores. Resection of cortical bone to expose spongy tissue that should grow into the adjacent open-celled implant (as is the case with the implant according to European Patent A 271,501) is not necessary.

Bars and/or spiny processes are preferably provided on both supporting surfaces of the implant which come to rest against the load bearing surfaces of the vertebrae in implantation and these bars and/or spiny processes are made of solid metal molded onto the solid core and their free ends project beyond the open-pore metal structure. When the implant is introduced between two vertebrae, the free ends of the bars and/or spiny processes project slightly into the surfaces of the vertebrae which are close together under the pressure of the spinal column and this yields a better positive and nonpositive primary fixation effect so the vertebrae are joined together so they cannot twist immediately after surgery.

The core of the implant preferably has a kidney-shaped peripheral contour and the free ends of the bars are designed with a cutting edge so the implant is anchored securely in the interspace between the adjacent vertebrae under the pressure of the spinal column.

The bars which are made of solid metal are molded onto the outer edge of the core in a preferred embodiment of this invention and form especially the ventral and dorsal sections of the periphery. In this form of the bars, the implant can be slipped between the vertebrae - in the direction of the bars. As an alternative, the bars can also be attached in the middle area of the core at right angles to the plane of the core, in which case the bars are aligned in the longitudinal direction of the kidney-shaped core. In all embodiments of this invention, an open-pore or open celled metal structure that has only the ends of the bars projecting beyond it is provided on the surfaces next to the vertebrae. The connective tissue which develops after the surgery is to grow into this open celled metal structure, thus stabilizing the torsional rigidity of the vertebra-implant-vertebra structure.

Preferably at least one socket or recess for attachment of a surgical instrument is shaped on the edge of the solid core. This socket is preferably arranged on one of the narrow sides of the kidney-shaped core.

Advantageous embodiments of this invention are characterized by the features of the subclaims.

Embodiments of this invention are explained in greater detail below with reference to the figures which show the following:
Figure 1 shows a perspective view of an implant according to this invention.
Figure 2 shows a cross section through the implant according to Figure 1 along line II-II.
Figure 3 shows a cross section along line III-III of Figure 1.
Figure 4 shows a side view of another embodiment of the implant.
Figure 5 shows a top view of the implant according to Figure 4.

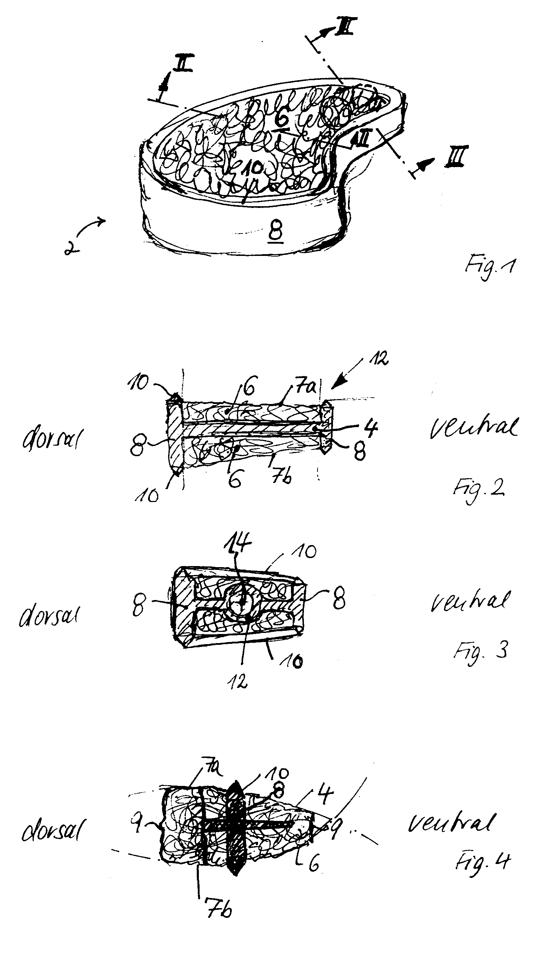

Figures 1 to 3 show a first embodiment of a disk-shaped implant 2 which serves to replace human disks. The implant 2 contains a core 4 made of a solid metal which is designed as a flat metal plate in the embodiment shown here and is provided with an open-pore or open-celled metal structure 6 on both sides. As an alternative, the core may have a wedge-shaped cross section tapering from the dorsal side to the ventral side. In addition the core may also have a slight curvature (not shown).

The disk-shaped core 4 has a kidney-shaped contour and has bars 8 that are molded on it and are also made of solid metal - shown at the edge of core 4 in the embodiment illustrated here - and the free ends 10 of these bars project over the open-pore metal structure 6. The bars 8 run around the entire disk-shaped core 4 and preferably have blade-shaped free ends that are supported against the hard bone surfaces of the vertebrae when inserted between adjacent vertebrae and are anchored in an adequately form fitting manner to prevent a relative movement between the implant and the vertebrae.

In the embodiment illustrated here, the cranial supporting surface 7a and the caudal supporting surface 7b of the implant are inclined slightly so that the entire implant tapers in a wedge shape from the dorsal side to the ventral side. Bar 8 on the dorsal section of the edge is designed to be higher and bar 8 on the ventral section of the periphery is designed to be lower accordingly, and the thickness of the open-pore metal structure 6 increases from the ventral bar 8 to the dorsal bar 8 on both sides of the flat core.

At least one socket 12 or a recess for attaching surgical instruments is provided on the edge of the solid core 4 so that surgical instruments can be inserted into the interior 14 of socket 12 and can be engaged there. Socket 12 is provided on a narrow side of the kidney-shaped core 4.

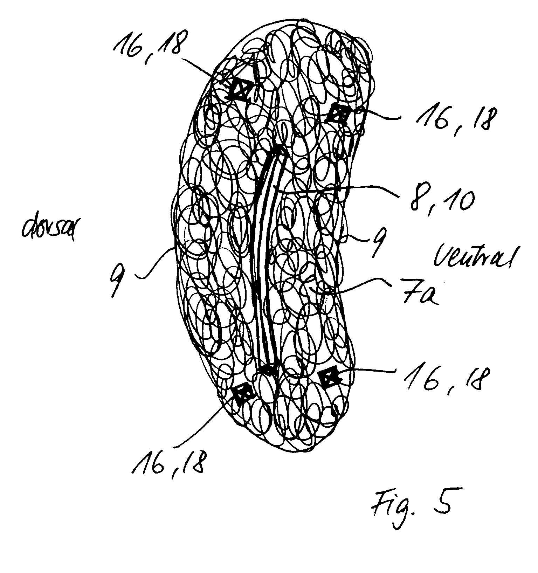

Figure 4 shows a side view, partially in section of another embodiment of implant 2. A disk-shaped core 4 made of solid metal that becomes thicker in a wedge shape from the ventral side to the dorsal side is provided with an open-pore metal structure on both sides and on its periphery and is shaped so it is an integral part of the core. A bar 8 running approximately at the mid-point between the dorsal edge and the ventral edge 9 of the implant with a kidney-shaped curvature in the longitudinal direction of the implant passes through the open-pore metal structure 6 on both sides. The open-pore metal structure 6 is symmetrical about 4 and the implant 2 increases in thickness in a wedge shape from the ventral side to the dorsal side. The cranial and caudal supporting surfaces 7a and 7b have a convex curvature in the ventral dorsal direction and optionally also in the lateral direction in order to assure a better fit with the bearing surfaces of the vertebrae which have a corresponding concave curvature. The free end 10 of bar 8 projects beyond supporting surfaces 7a and 7b and is tapered in a blade-shaped pattern.

Figure 5 shows a top view of the implant according to Figure 4. The kidney-shaped contour of the implant as well as the free ends 10 of bars 8 projecting out of the open-pore metal structure 6 and the free ends 18 of the optional spiny processes 16 can be seen clearly.

## Claims

1. Disk-shaped implant (2) for reinforcing adjacent vertebrae characterized by a disk-shaped core (4) made of solid metal having an open-pored or open-celled metal structure (6) provided on the surfaces of the core (4).

2. Disk-shaped implant according to Claim 1, characterized in that bars (8) are provided about the periphery of the implant to enclose supporting surfaces (7a, 7b) of the implant said supporting surfaces (7a, 7b) being formed by the open-pored or open-celled structure.

3. Disk-shaped implant according to Claim 2, characterized in that the bars (8) are molded on core (4) and their free ends (10) project above the supporting surfaces (7a, 7b) of the implant.

4. Disk-shaped implant according to one of the preceding claims, characterized in that the core (4) has a kidney-shaped peripheral contour.

5. Disk-shaped implant according to claims 2, 3 or 4, characterized in that the free ends (10) of the bars (8) are pointed.

6. Disk-shaped implant according to claims 2, 3, 4 or 5, characterized in that the bars (8) are molded on the periphery of core (4).

7. Disk-shaped implant according to claims 2, 3, 4, 5 or 6, characterized in that the bars (8) on core (4) form a continuous peripheral edge.

8. Disk-shaped implant according to claim 1, characterized in that bars (8) are molded on core (4) between the ventral and dorsal edges (9) and run longitudinally along the kidney-shaped core (4).

9. Disk-shaped implant according to claim 1, characterized in that the peripheral edge of core (4) has an open-pore or open-celled surface structure (6).

10. Disk-shaped implant according to one of the preceding claims, characterized in that at least one socket (12) or recess (14) for attaching surgical instruments is provided on the edge of the solid core (4).

11. Disk-shaped implant according to Claim 10, characterized in that the socket (12) or recess (14) extends from a narrow side of the kidney-shaped core (4) into the implant.

12. Disk-shaped implant according to Claim 11, characterized in that the implant tapers to form a wedge shape.

13. Disk-shaped implant according to one of the preceding claims, characterized in that the core (4) is planar and the bars (8) stand substantially at right angles to the plane of the core (5).

14. Disk-shaped implant according to one of the preceding Claims 1 to 12, characterized in that the core (4) tapers to form a wedge shape.

15. Disk-shaped implant according to one of the preceding claims, characterized in that the two supporting surfaces (7a, 7b) have a convex curvature.

16. Disk-shaped implant according to Claim 15, characterized in that the curvature of the supporting surfaces (7a, 7b) is provided in the ventral-dorsal direction and/or in the lateral direction.

17. Disk-shaped implant according to any preceding claim characterized in that the implant is provided with spiny processes (16).
